(19) Europäisches Patentamt
European Patent Office
Office européen des brevets

(11) **EP 4 164 564 B1**

(12) **EUROPEAN PATENT SPECIFICATION**

(45) Date of publication and mention
of the grant of the patent:
**01.10.2025 Bulletin 2025/40**

(21) Application number: **21732562.0**

(22) Date of filing: **08.06.2021**

(51) International Patent Classification (IPC):
***A61F 9/008*** (2006.01)

(52) Cooperative Patent Classification (CPC):
**A61F 9/00825; A61F 9/00834;** A61F 9/00827;
A61F 2009/0087; A61F 2009/00872;
A61F 2009/00887; A61F 2009/00897

(86) International application number:
**PCT/IB2021/055025**

(87) International publication number:
**WO 2021/255579 (23.12.2021 Gazette 2021/51)**

(54) **OPHTHALMIC LASER SYSTEMS WITH Z-DIRECTION MULTI-FOCAL OPTICS**

OPHTHALMISCHE LASERSYSTEME MIT MULTIFOKALER OPTIK IN Z-RICHTUNG

SYSTÈMES LASER OPHTALMIQUES À ÉLÉMENTS OPTIQUES MULTI-FOCAUX À DIRECTION Z

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2020 US 202063039769 P**

(43) Date of publication of application:
**19.04.2023 Bulletin 2023/16**

(73) Proprietor: **Alcon Inc.
1701 Fribourg (CH)**

(72) Inventor: **BOR, Zsolt
Lake Forest, California 92630 (US)**

(74) Representative: **Murgitroyd & Company
165-169 Scotland Street
Glasgow G5 8PL (GB)**

(56) References cited:
**WO-A1-2018/182946     WO-A1-2019/147952
US-A1- 2017 266 042**

**Description**

TECHNICAL FIELD

This present disclosure relates generally to ophthalmic laser systems and, more particularly, to ophthalmic laser systems with multi-focal optics.

BACKGROUND

Ophthalmic laser systems deliver laser pulses to focus spots along a scan pattern in a target. These laser systems have a variety of uses. For example, the systems may be used to perform a surgical procedure on ophthalmic tissue. The laser pulses create plasma or cavitation bubbles at the focus spots when the beam intensity or energy density exceeds a plasma or photo-disruption threshold. The pattern of bubbles can form surgical incisions or photo-disrupted regions.

As another example, ophthalmic laser systems may be used to adjust a light (or laser) adjustable lens (LAL). In cataract surgery, the cloudy natural lens is removed and replaced with an artificial intraocular lens (IOL). Pre-surgery eye measurements are used to calculate the power and type of IOL that will optimize vision after surgery. However, because of the limited accuracy of pre-surgical measurements and because eyes heal differently, it can be difficult to obtain the desired visual outcome.

A light adjustable lens can be adjusted after surgery to improve vision. The lens is made of a photo-sensitive material with refractive properties that can change in response to light. After the eye heals, the patient's vision is tested, and a laser system is used to scan light into the patient's eye to adjust the lens.

Reference is made to the documents US 2017/266042 A1, WO 2018/182946 A1 and WO 2019/147952 A1 which have been cited as relating to the state of the art.

BRIEF SUMMARY

The scope is in accordance with the appended claims. Surgical methods performed on the living tissue are not part of the claimed subject-matter.

In certain embodiments, an ophthalmic laser system comprises a laser source, multi-focal optics, scanners, delivery optics, and a computer. The laser source generates a laser beam of ultrashort laser pulses. The multi-focal optics multiplex the laser beam to yield focus spots in a target along a propagation axis of the laser beam. The scanners direct the laser beam in x, y, and z directions, where the z direction is defined by an optical axis of the laser system and the x and y directions are orthogonal to the z-direction. The delivery optics focus the laser beam within the target to form the focus spots in the target along the propagation axis of the laser beam. The computer instructs the scanners and the delivery optics to direct and to focus the focus spots at the target according to a scan pattern.

Embodiments may include none, one, some, or all of the following features.

The multi-focal optics comprise a diffractive optical element that multiplexes the laser beam to yield the focus spots along the propagation axis of the laser beam.

The multi-focal optics comprise a holographic optical element with an interference pattern with a high diffraction efficiency that yields the focus spots along the propagation axis of the laser beam.

The multi-focal optics comprises a computer-controlled spatial light modulator that modulates a feature of the laser beam to form the focus spots along the propagation axis of the laser beam.

At least two of the focus spots are spatially separated by a distance greater than the depth of focus of the laser beam.

The target comprising a lens for an eye. The lens may comprise an intraocular lens (IOL) for the eye or a contact lens for the eye. The computer may determine the scan pattern for the lens for hyperopia, myopia, or astigmatism correction of the eye.

The target comprises a cataractous lens of an eye. The computer instructs the scanners and the delivery optics to direct and to focus the focus spots to open a lens capsule with an incision, and emulsify the cataractous lens.

The target comprises a cornea of an eye. The computer instructs the scanners and the delivery optics to direct and to focus the focus spots to create an incision in the cornea.

In certain embodiments, an ophthalmic laser system comprises a laser source, multi-focal optics, scanners, delivery optics, and a computer. The laser source generates a laser beam of ultrashort laser pulses. The multi-focal optics multiplex the laser beam to yield focus spots in a target along a propagation axis of the laser beam. The target comprises a lens for an eye. The scanners direct the laser beam in x, y, and z directions, where the z direction is defined by an optical axis of the laser system and the x and y directions are orthogonal to the z-direction. The delivery optics focus the laser beam within the target to form the focus spots in the target along the propagation axis of the laser beam. The computer determines a scan pattern for hyperopia, myopia, or astigmatism correction of the eye, and instructs the scanners and the delivery optics to direct and to focus the focus spots at the target according to a scan pattern.

Embodiments may include none, one, some, or all of the following features.

The multi-focal optics comprise a diffractive optical element that multiplexes the laser beam to yield the focus spots along the propagation axis of the laser beam.

The multi-focal optics comprise a holographic optical element with an interference pattern with a high diffraction efficiency that yields the focus spots along the propagation axis of the laser beam.

The multi-focal optics comprise a computer-controlled spatial light modulator that modulates a feature of the laser beam to form the focus spots along the propagation axis of the laser beam.

**In** certain embodiments, a method for scanning a laser beam of an ophthalmic laser system comprises: generating, by a laser source, a laser beam of ultrashort laser pulses; multiplexing, by multi-focal optics, the laser beam to yield focus spots in a target along a propagation axis of the laser beam; directing, by scanners, the laser beam in x, y, and z directions; focusing, by delivery optics, the laser beam within the target to form the focus spots in the target along the propagation axis of the laser beam; and instructing, by a computer, the scanners and the delivery optics to direct and to focus the focus spots at the target according to a scan pattern.

Embodiments may include none, one, some, or all of the following features.

The method further comprises spatially separating at least two of the focus spots by a distance greater than the depth of focus of the laser beam.

The target comprises a lens for an eye. The lens may comprise an intraocular lens (IOL) for the eye or a contact lens for the eye. The method further comprises determining, by the computer, the scan pattern for the lens for hyperopia, myopia, or astigmatism correction of the eye.

BRIEF DESCRIPTION OF THE DRAWINGS

FIGURE 1 is a block diagram of an example ophthalmic surgical laser system that performs a procedure on a target;

FIGURE 2 illustrates example parts that may be used by the system of FIGURE 1;

FIGURES 3A and 3B illustrate examples of multi-focal diffractive optics that may be used by the system of FIGURE 1;

FIGURES 4A and 4B illustrate a relationship among the separation between focus spots F1 and F2, the cone angle of a portion of the beam that forms focus spot F2, and the energy loss due to the obscuration effect of focus spot F1 on focus spot F2; and

FIGURE 5 illustrates an example method for forming focus spots in a target that may be performed by the system of FIGURE 1.

DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

In general, the present disclosure relates to ophthalmic laser systems with multi-focal optics. In certain embodiments, an ophthalmic laser system includes multi-focal optics that multiplexes a laser beam to yield multiple (e.g., double, triple, or more) focus spots along the propagation axis of the beam. In this way, the effective laser repetition rate can be multiplied (e.g., doubled, tripled, or more) without facing the technical challenges of increasing the repetition rate of a laser source or of increasing the speed of the scanners. Additionally, the spatial separation between focus spots along the propagation axis may be selected to reduce or minimize shadowing effects that a bubble at a shallower focus spot may have on forming a bubble at a deeper depth. Accordingly, embodiments provide a solution for increasing the effective repetition rate of an ophthalmic laser system, resulting in decreased treatment time. These embodiments may be particularly useful for customizing femtosecond laser adjustable lenses (FLALs), which are intraocular lenses that comprise material with a refractive index that can be modified by femtosecond laser pulses.

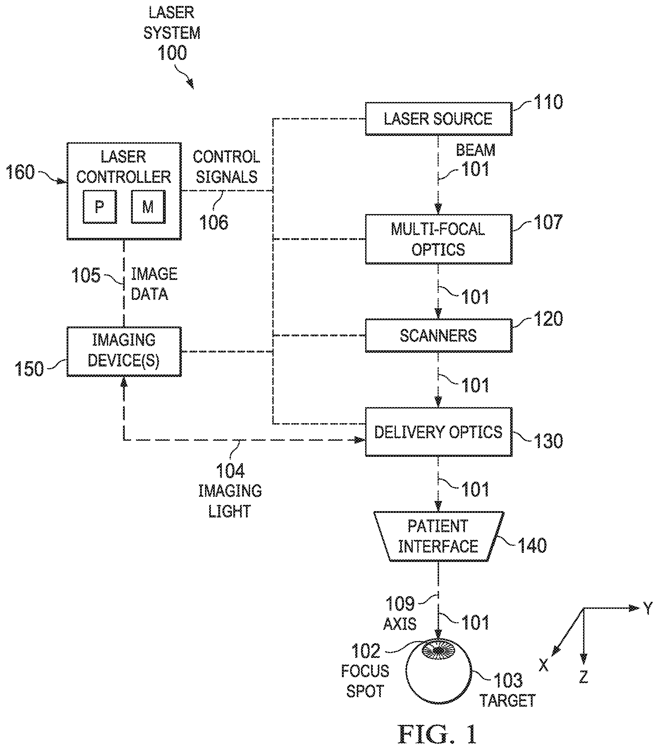

FIGURE 1 is a block diagram of an example ophthalmic surgical laser system 100 that performs a procedure on a target 103. The system 100 includes a laser source 110, multi-focal optics 107, scanners 120, delivery optics 130, a patient interface 140, an imaging device 150, and a laser controller 160. In an example of operation, laser source 110 generates a beam 101 of ultrashort laser pulses. Multi-focal optics 107 multiplexes beam 101 to yield multiple focus spots 102 along the propagation axis of the beam 101. Scanners 120 direct focus spots of beam 101 towards points of target 103. Delivery optics 130 focuses the scanned beam 101 through patient interface 140 to yield the focus spots 102 in target 103 along the propagation axis of the beam 101. Imaging device 150 generates images of target 103 during the procedure. Laser controller 160 controls laser source 110, multi-focal optics 107, scanners 120, delivery optics 130, and/or imaging device 150 to generate a scan pattern of spots in target 103. In the xyz-coordinate system of the example, the z-axis is defined by the propagation axis 109 of beam 101, and the xy-plane is orthogonal to z-axis.

System 100 includes optics. "Optics" refers to one or more optical elements that act on (e.g., transmit, reflect, refract, diffract, collimate, condition, shape, focus, modulate, and/or otherwise act on) beam 101. Examples of optical elements include a lens, prism, mirror, diffractive optical element (DOE), holographic optical element (HOE), and a spatial light modulator (SLM). A diffractive optical element typically has a microstructured surface relief profile that reshapes light to a different distribution by diffraction. Examples of diffractive optical elements include a beam-splitter, pattern generator, kinoform, beam shaper, and linear or circular grating. A holographic optical element is an optical element with an interference pattern produced using holographic imaging processes. Examples of a holographic optical elements include a lens, filter, beam splitter, or diffraction grating. A spatial light modulator is a computer-controlled device that modulates one or more features (e.g., the amplitude, phase, and/or polarization) of light waves in space and time. A spatial light modulator may have translucent (LCD) or reflective (LCOS) liquid crystal micro-displays.

In certain embodiments, laser source 110 comprises a laser engine capable of generating beam 101 of ultrashort laser pulses, e.g., pulses in the femtosecond, picosecond, or attosecond range. In certain variants, laser source 110 comprises a chirped pulse amplification (CPA) laser, which may include: an oscillator to generate femtosecond seed pulses; a stretcher to stretch the seed pulses by a factor of 10-1000 to the picosecond range; an amplifier to amplify the picosecond pulses; and a compressor to compress the duration of the amplified pulses back to the femtosecond range. In certain variants, laser source 110 comprises a cavity-dumped regenerative amplifier laser, which may include: an oscillator, stretcher-compressor, and optical amplifier. Examples of laser source 110 include a bulk laser, fiber laser, or hybrid laser.

In certain variants, the laser pulses generated by laser source 110 may have any suitable values for the following parameters, where example ranges of the values are as follows.

(1) <u>Pulse Duration</u>: 10 to 5000 femtoseconds (fs), such as 100 to 200, 200 to 300, 300 to 400, 400 to 500, 500 to 800, and/or 800 to 1000 fs. The pulse duration value may be selected according to application. For example, for cataract surgery, the value may be 400 to 800 fs. As another example, for adjusting a femtosecond laser adjustable lens (FLAL), the value may be 400 to 800 fs for certain lenses, or shorter, such as 10 to 300 fs, for other lenses.

(2) <u>Per-Pulse Energy</u>: 0.01 to 100 microjoule ($\mu$J), such as 0.1 to 30 $\mu$J.

(3) <u>Repetition Frequency</u>: 1 kilohertz (kHz) to 20 megahertz (MHz). The repetition frequency (or rate) value may be selected according to application. For example, for cataract surgery, the value may be 50 to 500 kHz or up to 2 MHz. As another example, for adjusting a FLAL, the value may be up to 10 MHz.

(4) <u>Spot Separation</u>: 0.01 to 10 micrometers ($\mu$m), such as 1 to 5 $\mu$m, in the xy-direction. For spot separation in the z-direction, the z-separation may be around or larger than the depth of focus of the laser beam.

(5) <u>Average Power of the Laser</u>: up to 3 watts (W). The average power of the laser (which is equal to the repetition rate times the energy of a single pulse) may be limited by safety standards. For example, the ANSI maximum possible exposure is approximately 3 W. This value depends on the focusing angle of the laser beam entering the eye.

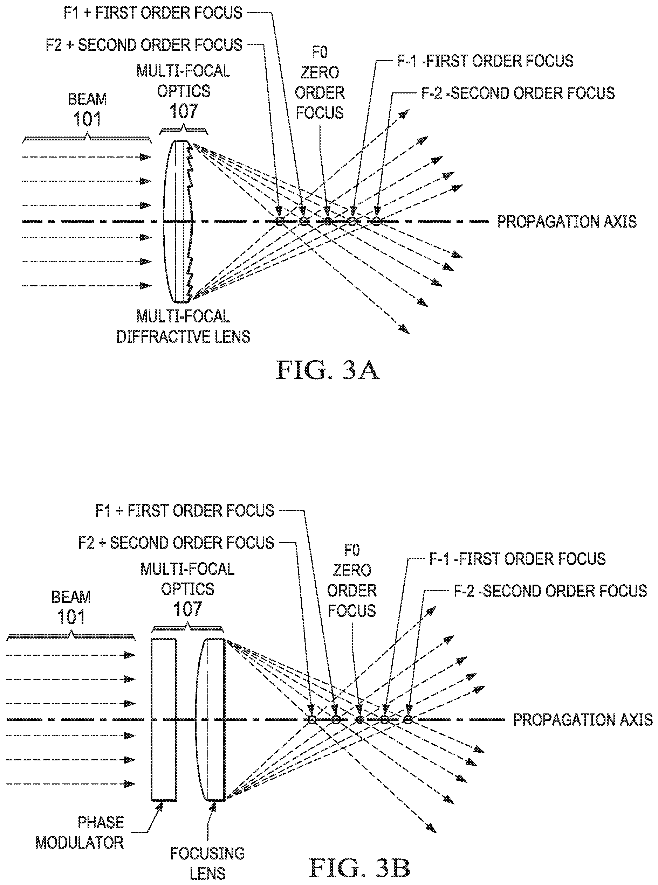

Multi-focal optics 107 described herein multiplex beam 101 to yield a plurality of focus spots 102 in a target along the propagation axis of beam 101. Multi-focal optics 107 may multiplex beam 101 by altering the pulses of beam 101 to yield multiple focus spots 102, e.g., by diffracting or refracting different portions of beam to different focus spots 102, or by modulating the amplitude, phase, and/or polarization of beam 101 to yield different focus spots 102. Examples of multi-focal optics 107 include a diffractive optical element, holographic optical element, and spatial light modulator. A diffractive optical element may have a microstructured surface relief profile or a pattern of different refractive indices that alters a laser beam to form multiple focus spots along the propagation axis of the laser beam. A holographic optical element may have an interference pattern with a high diffraction efficiency that forms the multiple focus spots. A spatial light modulator may modulate the amplitude, phase, and/or polarization of the laser beam to form the multiple focus spots, e.g., the modulator may be an electrically-addressed spatial light modulator that modulates the phase. Examples of multi-focal optics 107 are described in more detail with reference to FIGURES 3A and 3B.

Scanners 120 scan beam 101 to direct focus spots 102 of beam 101 towards points of target 103 in response to instructions from laser controller 160. Scanners 120 include any suitable combination of xy-scanner(s) and z-scanner(s). The optical axis of the laser system 100 defines the z-axis, and an xy-plane is orthogonal to the z-axis. An xy-scanner scans focus spot 102 of beam 101 in an xy-plane, while a z-scanner scans focus spot 102 of beam 101 in the z-direction parallel to the z-axis. Scanners 120 may include galvo scanners, which are computer controlled electromagnetic devices that rotate mirrors mounted at the ends of a rotary shaft. The mirror deflects beam 101 to scan the beam in the xy-plane. Scanners 120 may also include linear servomotors that scan beam 101 in the z-direction.

Delivery optics 130 focuses beam 101 to yield focus spots 102 in target 103 in response to instructions from laser controller 160. Delivery optics 130 may include a focusing objective lens, a beam expander, a birefringent lens, and other lenses to direct, collimate, condition, and/or focus the scanned beam 101 through patient interface 140 to focus spot 102 of target 103.

Patient interface 140 may attach to and immobilize target 103 during a laser procedure. Patient interface 140 may include, for example, a one or two-piece transparent applanation lens attached to a mount on delivery optics 130. The mount can provide a stable connection between the patient interface and delivery optics 130.

In certain embodiments, target 103 may comprise a particular type of artificial intraocular lens (IOL), a laser adjustable lens (LAL) (also known as "light adjustable lens"). A light adjustable lens is an artificial lens implanted during, e.g., cataract surgery. After the eye has healed, the refractive properties of the lens can be adjusted by directing beam 101 onto the lens from outside of the eye to form spots 102. A laser adjustable lens may be a femtosecond laser adjustable lens (FLAL), which comprises material with a refractive index that can be modified by femtosecond laser pulses. The laser pulses may modify the refractive index in any suitable manner. For example, the pulses may change the hydration level of the lens material (as well as that of the cornea). Increasing the hydration level decreases the refractive index, and decreasing hydration level increases the refractive index. As another example, the pulses may change the crosslinking of the lens material (or that of the cornea), which alters the refractive index.

In other embodiments, target 103 may comprise a contact lens that comprises material with a refractive index that can be modified by femtosecond laser pulses. The laser pulses may modify the refractive index in any suitable manner, e.g., the manners as described above with reference to FLALs. The refractive power and high order aberrations of the contact lens can be customized according the high order aberrations of the patient. In these embodiments, the contact lens is placed on a holder when modified by the laser pulses, i.e., the contact lens is not on an eye.

In yet other embodiments, target 103 may comprise an eye. A laser pulse may create a plasma or cavitation bubble in the eye at a focus spot 102 of beam 101 when the intensity or energy density exceeds a plasma or photo-disruption threshold of the eye. For example, in cataract surgery, focus spots 102 may be form incisions in the cornea and/or capsule to access the cataractous lens of the eye. Focus spots 102 may also emulsify the cataractous lens, and the multiple focus spots may decrease lens fragmentation time. As another example, in refractive surgery, focus spots 102 may be form incisions (e.g., a flap, a lenticule, or other incision) or other patterns in the cornea to change the refractive properties of the cornea.

Imaging device 150 receives imaging light 104 and generates real-time images of target 103 during a procedure. Imaging device 150 may generate image data 105 and send the data 105 to a laser controller 160. Examples of an imaging device 150 include a surgical microscope, video microscope, digital microscope, ophthalmoscope, optical coherence tomography (OCT) imaging system, and/or camera.

Laser controller 160 is a computer that comprises memory M storing instructions executable by a processor P to control pulsed laser source 110, multi-focal optics 107, scanners 120, delivery optics 130, and/or imaging devices 150. Typically, the processor of laser controller 160 comprises one or more CPUs (such as those manufactured by Intel, AMD, and others), microprocessors, field-programmable gate arrays (FPGAs), application-specific integrated circuits (ASICs), digital-signal processors (DSPs), or system-on-chip (SoC) processors communicatively coupled to memory. The memory may comprise a non-transitory computer-readable medium, and may include volatile or non-volatile memory including, magnetic media, optical media, random access memory (RAM), read-only memory (ROM), removable media, or analogous components. The memory may store software instructions executable by the processor to generate control signals 106 that control the operation of pulsed laser source 110, scanners 120, delivery optics 130, and imaging device 150.

In certain embodiments, laser controller 160 generates signals 106 to control parameters of beam 101 generated by pulsed laser source 110, such as a repetition rate, pulse length, and pulse energy. Laser controller 160 also generates signals 106 to instruct multi-focal optics 107, scanners 120, and/or delivery optics 130 to direct and focus spots 102 according to a scan pattern. A scan pattern may be any suitable two-dimensional or three-dimensional shape or pattern, including spiral, raster, zig-zag, circular, elliptical, or cylindrical patterns.

Laser controller 160 may determine the scan pattern according to the purpose of the operation. In certain embodiments, a scan pattern may be used to adjust the refractive properties of a light adjustable lens. For example, the scan pattern may form focus spots 102 in a light adjustable lens to change the refractive properties of the lens. Laser controller 160 may determine the scan pattern according to the type of correction. For hyperopia or myopia correction, the refractive properties may be changed to yield an intraocular lens that directs light onto the retina of the eye. For example, to treat hyperopia, the refractive index may be increased at in a central area and/or or decreased in a peripheral area. To treat myopia, the refractive index may be decreased in a central area and/or or increased in a peripheral area.

A central area may be described by a diameter that is a percentage of the total diameter of the lens, where the percentage has a value in the range of, e.g., 2 to 5, 5 to 10, 10 to 25, and/or 25 to 50 percent. For example, if the percentage is 10 percent, the central area is described by a diameter that is 10 percent of the total diameter of the lens. A peripheral area may be an annular region, where the outer ring may be described by a diameter r1 that is a percentage of the total diameter of the lens, and the inner ring may be described by a diameter d2 that is also a percentage of the total diameter of the lens, but d2 < d1. The percentages may have a value in the range of, e.g., 60 to 70, 70 to 80, 80 to 90, and/or 90 to 99 percent.

For astigmatism correction, focus spots 102 may be formed in a band across the lens. The band may be of any suitable size and shape to compensate for refractive errors of the eye, which may be determined by, e.g., an aberrometer or corneal topographer.

In other embodiments, a scan pattern may be used to perform a surgical procedure on an eye. For example, in cataract surgery, a scan pattern directs focus spots 102 to form incisions in the cornea and/or capsule to access the lens of the eye. A scan pattern may also direct focus spots 102 to open the lens capsule with a circular incision and emulsify the cataractous lens. As another example, in refractive surgery, a scan pattern directs focus spots 102 to form incisions (e.g., a flap, a lenticule, or other incision) or other pattern in the cornea to change the refractive properties of the cornea.

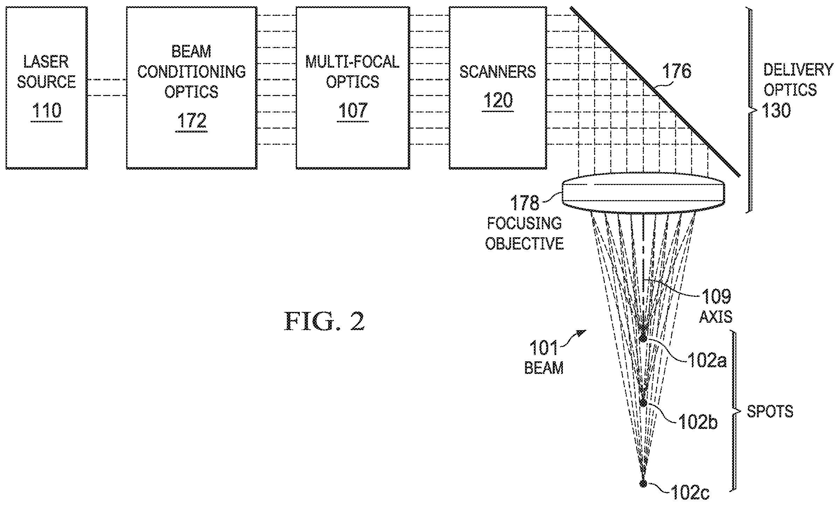

FIGURE 2 illustrates example parts that may be used by the system 100 of FIGURE 1. In the illustrated embodiment, system 100 includes laser source 110, beam conditioning optics 172, multi-focal optics 107, scanners 120, and delivery optics 130 that yield focus spots 102 (102a, 102b, 102c). In the illustrated example, delivery optics includes directing optics 176 and a focusing objective 178.

In the illustrated example, beam conditioning optics 172 conditions beam 101, such as expand and/or collimate beam 101. Beam conditioning optics 172 may include, e.g., an expander and/or collimator. Multi-focal optics 107 multiplexes beam 101 to yield a plurality of focus spots 102 in a target along propagation axis 109 of the beam 101. Directing optics 176 directs beam 101 towards focusing objective 178, which focuses beam 101 to focus spots 102. In the illustrated example, directing optics 176 may be a mirror that reflects beam 101 towards focusing objective 178. Note that even though directing optics 176 changes the direction of beam 101, the focus spots are still located along propagation axis 109 of the beam 101. In other examples, directing optics 176 may transmit or refract beam 101 or may be omitted.

FIGURES 3A and 3B illustrate examples of multi-focal diffractive optics 107 that may be used by the system 100 of FIGURE 1. FIGURE 3A illustrates multi-focal optics 107 that comprise a Fresnel lens with a diffractive pattern that yields focus spots 102: +2, +1, 0, -1, and -2.

FIGURE 3B illustrates multi-focal optics 107 that comprise a phase modulator (e.g., a phase plate with a diffractive pattern) and a focusing lens that yield focus spots 102: +2, +1, 0, -1, and -2. The phase modulator may be a diffractive optical element, a holographic optical element, or a spatial light modulator.

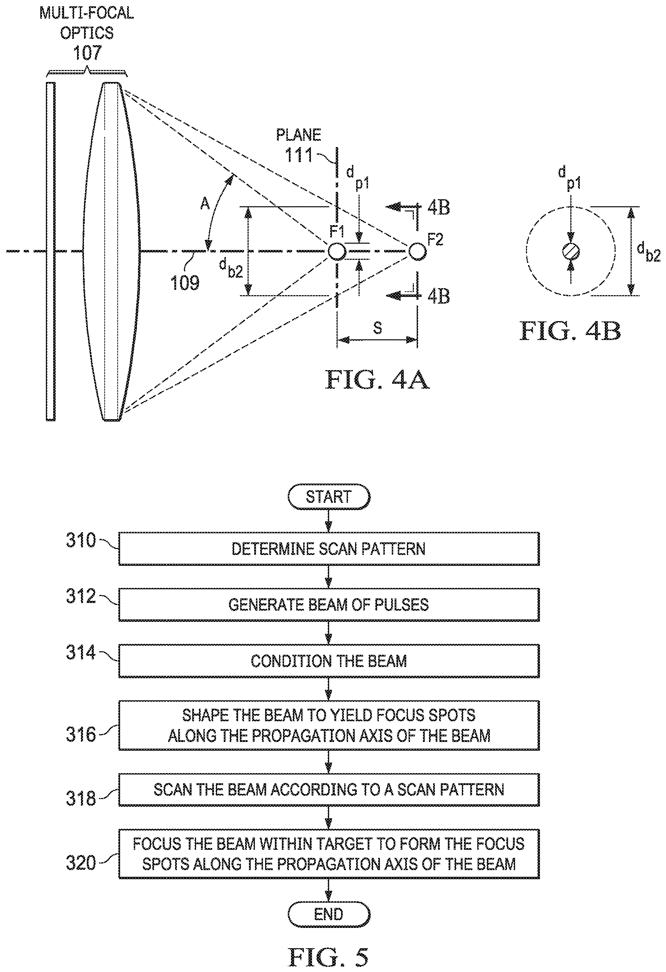

FIGURES 4A and 4B illustrate a relationship among the separation S between focus spots 102 (F1, F2) along propagation axis 109, the cone angle A of a portion of beam 101 that forms focus spot F2, and the energy loss due to the obscuration effect of focus spot F1 on focus spot F2. FIGURE 4A shows a view of multi-focal optics 107 and focus spots F1 and F2 along propagation axis 109. FIGURE 4B illustrates a view of plane 111 from focus spot F2.

In certain targets 103, e.g., where target 103 is a part of an eye, the plasma bubble formed by focus spot F1 may obscure the beam energy that is directed towards focus spot F2, yielding energy loss at focus spot F2. In some targets 103, e.g., where target 103 is a light adjustable lens, no plasma bubble is formed by focus spot F1, so this type of energy loss is not a concern.

Although the parameters of the illustrated example may have any suitable values, specific values have been assigned to more easily describe the relationship. In the example, the portion of beam that forms focus spot F2 forms a cone with a cone angle A of any suitable value, e.g., 0.1 to 0.2 radian, such as 0.15 radian. Separation S between focus spots F1 and F2 may have any suitable value, e.g., the z-separation may be larger than the depth of focus of the laser beam, such as 5 to 50, 50 to 100, 100 to 300, 300 to 500, and/or greater than 500 micrometers ($\mu$m), such as 200 $\mu$m. The diameter $d_{p1}$ of plasma bubble formed by focus spot F1 may be any suitable value, e.g., 2 to 5 $\mu$m, such as 3 $\mu$m.

In the illustrated example, focus spot F1 is closer to delivery optics 130 than focus spot F2 is to delivery optics 130, i.e., focus spot F1 is shallower than focus spot F2 or focus spot F2 deeper than focus spot F1. In certain situations, the plasma bubble formed by focus spot F1 may obscure the beam energy directed towards focus spot F2, yielding energy loss at focus spot F2. In the situations, the separation S between focal spots F1 and F2 may be selected to make negligible the energy loss caused by the obscuration effect of focus spot F1 on focus spot F2. Generally, increasing the separation S between focal spots F1 and F2 and/or increasing the cone angle A of the beam that forms focus spot F2 reduces the energy loss caused by the obscuration effect of focus spot F1 on focus spot F2.

The diameter $d_{b2}$ of the cone that forms focus spot F2, measured at the plane 111 orthogonal to propagation axis 109 where focus spot F1 intersects propagation axis 109, can be calculated from angle A and separation S:

$$d_{b2} = 2 \text{ x angle A x separation S} = 2 \text{ x } 0.15 \text{ x } 200 \text{ } \mu m = 60 \text{ } \mu m$$

The amount of obscuration may be measured by an obscuration ratio R:

$$R = (d_{p1}/d_{b2})^2 = (3 \text{ } \mu m/60 \text{ } \mu m)^2 = 1/400 = 0.0025 = 0.25 \text{ \%}$$

The energy loss $E_L$ may be calculated from obscuration ratio R:

$$E_L = R = 0.25\%$$

In certain embodiments such as in cataract or refractive surgery, a 0.25 % energy loss may be regarded as acceptable. A maximum acceptable energy loss P may depend on the type of surgery.

FIGURE 5 illustrates an example method for forming focus spots 102 in a target 103 that may be performed by system 100 of FIGURE 1. Focus spots 102 are formed along propagation axis 109 of laser beam 101.

The method starts at step 310, where system 100 determines a scan pattern. The scan pattern may be used to adjust a light adjustable lens or perform a surgical procedure in ophthalmic tissue (e.g., lens or cornea). In certain embodiments, laser controller 106 determines a scan pattern with a cone angle A and separation S between focus spots that satisfies a maximum acceptable energy loss P, as described with reference to FIGURE 4.

Laser source 110 generates laser beam 101 at step 312. Beam conditioning optics 172 conditions beam 101 at step 314. Multi-focal options 107 multiplexes beam 101 at step 316 to yield focus spots along propagation axis 109 of beam 101. Scanners 120 scan beam 101 according to the scan pattern at step 318. Delivery optics 130 focuses beam 101 at step 320 to form focus spots 102 in target 103. The method then ends.

A component (such as laser controller 160) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface (e.g., a Graphical User Interface (GUI)) is a type of interface that a user can utilize to interact with a computer. Examples of user interfaces include a display, touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by the electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

To aid the Patent Office and readers in interpreting the claims, Applicants note that they do not intend any of the claims or claim elements to invoke 35 U.S.C. §112(f), unless the words "means for" or "step for" are explicitly used in the particular claim. Use of any other term (e.g., "mechanism," "module," "device," "unit," "component," "element," "member," "apparatus," "machine," "system," "processor," or "controller") within a claim is understood by the applicants to refer to structures known to those skilled in the relevant art and is not intended to invoke 35 U.S.C. §112(f).

## Claims

1. An ophthalmic laser system (100), comprising:

   a laser source (110) configured to generate a laser beam (101) of ultrashort laser pulses;
   multi-focal optics (107) configured to multiplex the laser beam to yield a plurality of focus spots in a target along a propagation axis of the laser beam;
   a plurality of scanners (120) configured to direct the laser beam in x, y, and z directions, the z direction defined by an optical axis of the laser system, the x and y directions orthogonal to the z-direction;
   delivery optics (130) configured to focus the laser beam within the target to form the plurality of focus spots in the target along the propagation axis of the laser beam (101); and

a computer (160) configured to instruct the scanners and the delivery optics to direct and to focus the plurality of focus spots (102) at the target according to a scan pattern; and

wherein the focus spots are spatially separated by a distance greater than the depth of focus of the laser beam.

2. The ophthalmic laser system of Claim 1, the multi-focal optics (107) comprising a diffractive optical element, DOE, that multiplexes the laser beam to yield the plurality of focus spots along the propagation axis of the laser beam (101).

3. The ophthalmic laser system of Claim 1, the multi-focal optics (107) comprising a holographic optical element, HOE, with an interference pattern with a high diffraction efficiency that yields the plurality of focus spots along the propagation axis of the laser beam (101).

4. The ophthalmic laser system of Claim 1, the multi-focal optics (107) comprising a computer-controlled spatial light modulator, SLM, that modulates a feature of the laser beam to form the plurality of focus spots along the propagation axis of the laser beam.

5. The ophthalmic laser system of Claim 1, the computer configured to determine the scan pattern according to the target, wherein in the event that the target is a lens for an eye, the computer is configured to instruct the scanners and the delivery optics to direct and to focus the focus spots to the lens.

6. The ophthalmic laser system of Claim 5, wherein in the event that the lens comprises an intraocular lens (IOL) for the eye, the computer is configured to determine the scan pattern for the lens for hyperopia, myopia, or astigmatism correction of the eye.

7. The ophthalmic laser system of Claim 5, wherein in the event that the lens comprises a contact lens for the eye, the computer is configured to determine the scan pattern for the lens for hyperopia, myopia, or astigmatism correction of the eye.

8. The ophthalmic laser system of Claim 1:

the computer configured to determine the scan pattern according to the target, wherein in the event that the target comprises a cataractous lens of an eye,
the computer configured to instruct the scanners and the delivery optics to direct and to focus the plurality of focus spots to:

open a lens capsule with an incision; and
emulsify the cataractous lens.

9. The ophthalmic laser system of Claim 1:
the computer configured to determine the scan pattern according to the target, wherein in the event that the target comprises a cornea of an eye, the computer is configured to instruct the scanners and the delivery optics to direct and to focus the plurality of focus spots to create an incision in the cornea.

10. The ophthalmic laser system (100) as claimed in claim 1, the target comprising a lens for an eye; and wherein
the computer (160) is configured to:

determine a scan pattern for hyperopia, myopia, or astigmatism correction of the eye; and
instruct the scanners and the delivery optics to direct and to focus the plurality of focus spots according to the scan pattern.

11. The ophthalmic laser system of Claim 10, the multi-focal optics (107) comprising a diffractive optical element that multiplexes the laser beam to yield the plurality of focus spots along the propagation axis of the laser beam.

12. The ophthalmic laser system of Claim 10, the multi-focal optics (107) comprising a holographic optical element with an interference pattern with a high diffraction efficiency that yields the plurality of focus spots along the propagation axis of the laser beam.

13. The ophthalmic laser system of Claim 10, the multi-focal optics (107) comprising a computer-controlled spatial light modulator that modulates a feature of the laser beam to form the plurality of focus spots along the propagation axis of the laser beam.

14. A method for scanning a laser beam (101) of an ophthalmic laser system (100) as claimed in claim 1, comprising:

   generating, by a laser source (110), a laser beam of ultrashort laser pulses;
   multiplexing, by multi-focal optics (107), the laser beam to yield a plurality of focus spots in a target along a propagation axis of the laser beam, wherein the target consists of a non-implanted intraocular lens (IOL) or a contact lens;
   directing, by a plurality of scanners (120), the laser beam in x, y, and z directions, the z direction defined by an optical axis of the laser system, the x and y directions orthogonal to the z-direction;
   focusing, by delivery optics (130), the laser beam within the target to form the plurality of focus spots in the target along the propagation axis of the laser beam; and
   instructing, by a computer (160), the scanners (120) and the delivery optics (130) to direct and to focus the plurality of focus spots according to a scan pattern; and
   further comprising:
   spatially separating at least two of the focus spots (102) by a distance greater than the depth of focus of the laser beam.

**Patentansprüche**

1. Ophthalmisches Lasersystem (100), umfassend:

   eine Laserquelle (110), die dazu ausgelegt ist, einen Laserstrahl (101) mit ultrakurzen Laserimpulsen zu generieren;
   Multifokaloptik (107), die dazu ausgelegt ist, den Laserstrahl zu multiplexen, um mehrere Fokuspunkte in einem Ziel entlang einer Ausbreitungsachse des Laserstrahls zu erhalten;
   mehrere Scanner (120), die dazu ausgelegt sind, den Laserstrahl in x-, y- und z-Richtung auszurichten, wobei die z-Richtung durch eine optische Achse des Lasersystems definiert ist, wobei die **x-** und y-Richtung orthogonal zu der z-Richtung sind;
   Führungsoptik (130), die dazu ausgelegt ist, den Laserstrahl innerhalb des Ziels zu fokussieren, um die mehreren Fokuspunkte in dem Ziel entlang der Ausbreitungsachse des Laserstrahls (101) auszubilden; und
   einen Computer (160), der dazu ausgelegt ist, die Scanner und die Führungsoptik anzuweisen, die mehreren Fokuspunkte (102) gemäß einem Scanmuster auf das Ziel auszurichten und zu fokussieren; und
   wobei die Fokuspunkte räumlich durch einen Abstand getrennt sind, der größer als die Fokustiefe des Laserstrahls ist.

2. Ophthalmisches Lasersystem nach Anspruch 1, wobei die Multifokaloptik (107) ein diffraktives optisches Element, DOE, umfasst, das den Laserstrahl multiplext, um die mehreren Fokuspunkte entlang der Ausbreitungsachse des Laserstrahls (101) zu erhalten.

3. Ophthalmisches Lasersystem nach Anspruch 1, wobei die Multifokaloptik (107) ein holographisches optisches Element, HOE, mit einem Interferenzmuster mit einer hohen Diffraktionseffizienz umfasst, das die mehreren Fokuspunkte entlang der Ausbreitungsachse des Laserstrahls (101) erhält.

4. Ophthalmisches Lasersystem nach Anspruch 1, wobei die Multifokaloptik (107) einen computergesteuerten räumlichen Lichtmodulator, SLM, umfasst, der ein Merkmal des Laserstrahls moduliert, um die mehreren Fokuspunkte entlang der Ausbreitungsachse des Laserstrahls auszubilden.

5. Ophthalmisches Lasersystem nach Anspruch 1, wobei der Computer dazu ausgelegt ist, das Scanmuster gemäß dem Ziel zu bestimmen, wobei, falls das Ziel eine Linse für ein Auge ist, der Computer dazu ausgelegt ist, die Scanner und die Führungsoptik anzuweisen, die Fokuspunkte auf die Linse auszurichten und zu fokussieren.

6. Ophthalmisches Lasersystem nach Anspruch 5, wobei, falls die Linse eine Intraokularlinse (IOL) für das Auge umfasst, der Computer dazu ausgelegt ist, das Scanmuster für die Linse für Hyperopie, Myopie oder Astigmatismuskorrektur des Auges zu bestimmen.

7. Ophthalmisches Lasersystem nach Anspruch 5, wobei, falls die Linse eine Kontaktlinse für das Auge umfasst, der Computer dazu ausgelegt ist, das Scanmuster für die Linse für Hyperopie, Myopie oder Astigmatismuskorrektur des Auges zu bestimmen.

8. Ophthalmisches Lasersystem nach Anspruch 1:

   wobei der Computer dazu ausgelegt ist, das Scanmuster gemäß dem Ziel zu bestimmen, wobei, falls das Ziel eine kataraktöse Linse eines Auges umfasst,
   der Computer dazu ausgelegt ist, die Scanner und die Führungsoptik anzuweisen, die mehreren Fokuspunkte auszurichten und zu fokussieren, zum:
   Öffnen einer Linsenkapsel mit einem Einschnitt und Emulgieren der kataraktösen Linse.

9. Ophthalmisches Lasersystem nach Anspruch 1:
   wobei der Computer dazu ausgelegt ist, das Scanmuster gemäß dem Ziel zu bestimmen, wobei, falls das Ziel eine Hornhaut eines Auges umfasst, der Computer dazu ausgelegt ist, die Scanner und die Führungsoptik anzuweisen, die mehreren Fokuspunkte auszurichten und zu fokussieren, um einen Einschnitt in der Hornhaut zu erzeugen.

10. Ophthalmisches Lasersystem (100) nach Anspruch 1, wobei das Ziel eine Linse für ein Auge umfasst und wobei
    der Computer (160) ausgelegt ist zum:

    Bestimmen eines Scanmusters für Hyperopie, Myopie oder Astigmatismuskorrektur des Auges und
    Anweisen der Scanner und Führungsoptik, die mehreren Fokuspunkte gemäß dem Scanmuster auszurichten und zu fokussieren.

11. Ophthalmisches Lasersystem nach Anspruch 10, wobei die Multifokaloptik (107) ein diffraktives optisches Element umfasst, das den Laserstrahl multiplext, um die mehreren Fokuspunkte entlang der Ausbreitungsachse des Laserstrahls zu erhalten.

12. Ophthalmisches Lasersystem nach Anspruch 10, wobei die Multifokaloptik (107) ein holografisches optisches Element mit einem Interferenzmuster mit einer hohen Diffraktionseffizienz umfasst, das die mehreren Fokuspunkte entlang der Ausbreitungsachse des Laserstrahls erhält.

13. Ophthalmisches Lasersystem nach Anspruch 10, wobei die Multifokaloptik (107) einen computergesteuerten räumlichen Lichtmodulator umfasst, der ein Merkmal des Laserstrahls moduliert, um die mehreren Fokuspunkte entlang der Ausbreitungsachse des Laserstrahls auszubilden.

14. Verfahren zum Scannen eines Laserstrahls (101) eines ophthalmischen Lasersystems (100) nach Anspruch 1, umfassend:

    Erzeugen eines Laserstrahls aus ultrakurzen Laserimpulsen durch eine Laserquelle (110);
    Multiplexen des Laserstrahls durch Multifokaloptik (107), um mehrere Fokuspunkte in einem Ziel entlang einer Ausbreitungsachse des Laserstrahls zu erhalten, wobei das Ziel aus einer nicht implantierten Intraokularlinse (IOL) oder einer Kontaktlinse besteht;
    Ausrichten des Laserstrahls in x-, y- und z-Richtung durch mehrere Scanner (120), wobei die z-Richtung durch eine optische Achse des Lasersystems definiert ist, wobei die x- und y-Richtung orthogonal zu der z-Richtung sind;
    Fokussieren des Laserstrahls innerhalb des Ziels durch Führungsoptik (130), um die mehreren Fokuspunkte in dem Ziel entlang der Ausbreitungsachse des Laserstrahls auszubilden; und
    Anweisen der Scanner (120) und Führungsoptik (130) durch einen Computer (160), die mehreren Fokuspunkte gemäß einem Scanmuster zu lenken und zu fokussieren; und
    ferner umfassend:
    räumliches Trennen von mindestens zwei der Fokuspunkte (102) um einen Abstand, der größer als die Fokustiefe des Laserstrahls ist.

**Revendications**

1. Système laser ophtalmique (100), comprenant :

   une source laser (110) configurée pour générer un faisceau laser (101) d'impulsions laser ultra-courtes ;
   des éléments optiques multifocaux (107) configurés pour multiplexer le faisceau laser afin de produire une pluralité de points de focalisation dans une cible le long d'un axe de propagation du faisceau laser ;
   une pluralité de dispositifs de balayage (120) configurés pour diriger le faisceau laser dans les directions x, y et z, la direction z étant définie par un axe optique du système laser, les directions x et y étant orthogonales à la direction z ;
   des éléments optiques de distribution (130) configurés pour focaliser le faisceau laser à l'intérieur de la cible afin de former la pluralité de points de focalisation dans la cible le long de l'axe de propagation du faisceau laser (101) ; et
   un ordinateur (160) configuré pour ordonner aux dispositifs de balayage et aux éléments optiques de distribution de diriger et de focaliser la pluralité de points de focalisation (102) au niveau de la cible selon un motif de balayage ; et
   les points de focalisation étant séparés spatialement d'une distance supérieure à la profondeur de focalisation du faisceau laser.

2. Système laser ophtalmique selon la revendication 1, les éléments optiques multifocaux (107) comprenant un élément optique diffractif, DOE, qui multiplexe le faisceau laser pour produire la pluralité de points de focalisation le long de l'axe de propagation du faisceau laser (101).

3. Système laser ophtalmique selon la revendication 1, les éléments optiques multifocaux (107) comprenant un élément optique holographique, HOE, avec un motif d'interférence avec une efficacité de diffraction élevée qui produit la pluralité de points de focalisation le long de l'axe de propagation du faisceau laser (101).

4. Système laser ophtalmique selon la revendication 1, les éléments optiques multifocaux (107) comprenant un modulateur spatial de lumière commandé par ordinateur, SLM, qui module une caractéristique du faisceau laser pour former la pluralité de points de focalisation le long de l'axe de propagation du faisceau laser.

5. Système laser ophtalmique selon la revendication 1, l'ordinateur étant configuré pour déterminer le motif de balayage selon la cible, dans le cas où la cible est un cristallin pour un œil, l'ordinateur étant configuré pour ordonner aux dispositifs de balayage et aux éléments optiques de distribution de diriger et de focaliser les points de focalisation sur le cristallin.

6. Système laser ophtalmique selon la revendication 5, dans le cas où le cristallin comprend un cristallin intraoculaire (IOL) pour l'œil, l'ordinateur étant configuré pour déterminer le motif de balayage pour le cristallin pour la correction de l'hypermétropie, de la myopie ou de l'astigmatisme de l'œil.

7. Système laser ophtalmique selon la revendication 5, dans le cas où le cristallin comprend un verre de contact pour l'œil, l'ordinateur étant configuré pour déterminer le motif de balayage pour le cristallin pour la correction de l'hypermétropie, de la myopie ou de l'astigmatisme de l'œil.

8. Système laser ophtalmique selon la revendication 1 :

   le calculateur étant configuré pour déterminer le motif de balayage en fonction de la cible, dans le cas où la cible comprend un cristallin cataracté d'un œil,
   l'ordinateur étant configuré pour ordonner aux dispositifs de balayage et aux éléments optiques de distribution de diriger et de focaliser la pluralité de points de focalisation pour :
   ouvrir une capsule de cristallin avec une incision ; et émulsionner le cristallin cataracté.

9. Système laser ophtalmique selon la revendication 1 :
   l'ordinateur étant configuré pour déterminer le motif de balayage en fonction de la cible, dans le cas où la cible comprend une cornée d'un œil, l'ordinateur étant configuré pour ordonner aux dispositifs de balayage et aux éléments optiques de distribution de diriger et de focaliser la pluralité de points de focalisation afin de créer une incision dans la cornée.

10. Système laser ophtalmique (100) selon la revendication 1, la cible comportant un cristallin pour un œil ; et

l'ordinateur (160) étant configuré pour :

déterminer un motif de balayage pour la correction de l'hypermétropie, de la myopie ou de l'astigmatisme de l'œil ; et

ordonner aux dispositifs de balayage et aux éléments optiques de distribution de diriger et de focaliser la pluralité de points de focalisation en fonction du motif de balayage.

11. Système laser ophtalmique selon la revendication 10, les éléments optiques multifocaux (107) comprenant un élément optique diffractif qui multiplexe le faisceau laser pour produire la pluralité de points de focalisation le long de l'axe de propagation du faisceau laser.

12. Système laser ophtalmique selon la revendication 10, les éléments optiques multifocaux (107) comprenant un élément optique holographique comportant un motif d'interférence avec une efficacité de diffraction élevée qui produit la pluralité de points de focalisation le long de l'axe de propagation du faisceau laser.

13. Système laser ophtalmique selon la revendication 10, les éléments optiques multifocaux (107) comprenant un modulateur spatial de lumière commandé par ordinateur qui module une caractéristique du faisceau laser pour former la pluralité de points de focalisation le long de l'axe de propagation du faisceau laser.

14. Procédé de balayage d'un faisceau laser (101) d'un système laser ophtalmique (100) selon la revendication 1 comprenant :

la génération par une source laser (110) d'un faisceau laser d'impulsions laser ultra-courtes,
le multiplexage par des éléments optiques multifocaux (107) du faisceau laser pour produire une pluralité de points de focalisation dans une cible le long d'un axe de propagation du faisceau laser, la cible étant constituée d'un cristallin intraoculaire (IOL) non implantée ou d'un verre de contact ;
l'orientation, par une pluralité de dispositifs de balayage (120), du faisceau laser dans des directions x, y et z, la direction z étant définie par un axe optique du système laser, les directions x et y étant orthogonales à la direction z ;
la focalisation, par des éléments optiques de distribution (130), du faisceau laser à l'intérieur de la cible pour former la pluralité de points de focalisation dans la cible le long de l'axe de propagation du faisceau laser ; et
l'instruction, par un ordinateur (160), aux dispositifs de balayage (120) et aux éléments optiques de distribution (130) de diriger et de focaliser la pluralité de points de focalisation selon un motif de balayage ; et
comprenant en outre :
la séparation spatiale d'au moins deux des points de focalisation (102) d'une distance supérieure à la profondeur de focalisation du faisceau laser.

FIG. 1

FIG. 2

F1 + FIRST ORDER FOCUS

F2 + SECOND ORDER FOCUS

MULTI-FOCAL
OPTICS
107

BEAM
101

F0
ZERO
ORDER
FOCUS

F-1 -FIRST ORDER FOCUS

F-2 -SECOND ORDER FOCUS

PROPAGATION AXIS

MULTI-FOCAL
DIFFRACTIVE LENS

## FIG. 3A

F1 + FIRST ORDER FOCUS

F2 + SECOND ORDER FOCUS

MULTI-FOCAL
OPTICS
107

BEAM
101

F0
ZERO
ORDER
FOCUS

F-1 -FIRST ORDER FOCUS

F-2 -SECOND ORDER FOCUS

PROPAGATION AXIS

PHASE
MODULATOR

FOCUSING
LENS

## FIG. 3B

FIG. 4A

FIG. 4B

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017266042 A1
- WO 2018182946 A1
- WO 2019147952 A1